Europäisches Patentamt

European Patent Office

Office européen des brevets

(19)

(11) Veröffentlichungsnummer: **0 001 994**
**B1**

(12)

# EUROPÄISCHE PATENTSCHRIFT

(21) Anmeldenummer: **78101240.6**

(22) Anmeldetag: **27.10.78**

(51) Int. Cl.³: **C 07 C 103/38,**
**C 07 C 102/00,**
**C 07 C 103/34**

(54) Verfahren zur Herstellung von Acetoacetylaminobenzolen

(30) Priorität: **04.11.77 DE 2749327**

(43) Veröffentlichungstag der Anmeldung:
**30.05.79 Patentblatt 79/11**

(45) Bekanntmachung des Hinweises auf die Patenterteilung:
**17.09.80 Patentblatt 80/19**

(84) Benannte Vertragsstaaten:
**BE CH DE FR GB**

(56) Entgegenhaltungen:
**DE - B - 2 518 984**
**DE - A - 2 519 036**
**GB - A - 962 227**

(73) Patentinhaber: **HOECHST
AKTIENGESELLSCHAFT Postfach 80 03 20
D - 6230 Frankfurt/Main 80 (DE)**

(72) Erfinder: **Berthold, Rüdiger, Dr.
Geierfeld 55
D - 6232 Bad Soden am Taunus (DE)
Tronich, Wolfgang, Dr.
Martin-Wohmann-Strasse 27
D - 6238 Hofheim am Taunus (DE)**

Verfahren zur Herstellung von Acetoacetylaminobenzolen

Aus der DE-PS 2 518 984 ist ein Verfahren zur Herstellung von N-Acetoacetyl-2,5-dimethoxy-4-chloranilid bekannt, bei dem man 2,5-Dimethoxy-4-chloranilin in Wasser suspendiert, mit Diketen umsetzt, und wobei das Diketen gleich zu Beginn der Reaktion auf einmal zugegeben wird. Vorzugsweise werden bei dieser Umsetzung anorganische oder insbesondere organische Säuren zugesetzt, zweckmässig pro Mol Anilin 0,1 bis 1 Mol Säure.

Aus der DE-OS 25 19 036 ist es bekannt, Acetoacetylarylamide durch Umsetzung von primären aromatischen Aminen mit Diketen in Wasser dadurch herzustellen, dass man die Umsetzung bei einer Temperatur durchführt, bei der das Verfahrensprodukt nicht im festen Aggregatzustand vorliegt. Vorsugsweise wird diese Umsetzung in wässriger Essigsäure einer Konzentration von bis etwa 60 Gew.-% durchgeführt.

Aus der GB-PS 962 227 ist es bekannt. Acetoacetylarylamide durch Umsetzung der Arylamine mit Diketen in Toluol unter Zusatz von tertiären Aminen als Katalysator herzustellen.

Es wurde nun gefunden, dass bei drei speziell substituierten Anilinen die Umsetzung besonders gut verläuft und zu einem hochreinen Produkt führt, wenn als Reaktionsmedium ein inertes organisches Lösemittel verwendet wird und neben den katalytischen Säuremengen auch Katalystische Mengen an Wasser vorhanden sind.

Gegenstand der Erfindung ist somit ein Verfahren zur Herstellung von 1-Acetoacetylamino-2,5-dimethoxy-4-chlorbenzol, 1-Acetoacetylamino-2,4-dimethylbenzol und 1-Acetoacetylamino-2-methyl-5-chlorbenzol aus Diketen und dem entsprechenden Anilin in Gegenwart von Wasser unter Zusatz katalytischer Säuremengen, das dadurch gekennzeichnet ist, dass man das Anilin in einem inerten organischen Löse- oder Verdünnungsmittel in Gegenwart von 0,1 bis 6%, bezogen auf das Gewicht des Anilins, Wasser umsetzt.

Bevorzugt werden 0,1 bis 3%, insbesondere 0,15 bis 0,35%, Wasser eingesetzt. Bevorzugte Löse- oder Verdünnungsmittel sind Kohlenwasserstoffe wie Petroläther, Cyclohexan, Benzol, Toluol, Xylol oder technische Gemische solcher Lösemittel, wie sie beispielsweise unter dem Handelsnamen "Solventnaphtha" vertrieben werden. Es können aber auch inerte Chlorkohlenwasserstoffe, beispielsweise Chloraromaten verwendet werden. Bevorzugt sind, insbesondere beim Einsatz technischer feuchter Aminqualitäten, Löse- und Verdünnungsmittel, die mit Wasser ein Azeotrop bilden und somit ein einfaches Auskreisen des Wassers vor der Umsetzung mit dem Diketen erlauben, wie Toluol oder Xylol. Es stellt deshalb eine bevorzugte Ausführungsform dar, das Amin mit einem solchen Lösemittel, das mit Wasser ein Azeotrop bildet, zu trocknen und anschliessend die gewünschten Wassermengen zuzusetzen. Es ist jedoch auch möglich, ein technisches Anilin einzusetzen, das geringe Mengen Wasser enthält, und dann den erfindungsgemässen Bereich an Wasser einzustellen.

Grössere als die erfindungsgemässen Mengen an Wasser führen zu einer Qualitätseinbusse des Produktes und erfordern eine Aufarbeitung des anfallenden Abwassers. Als Säure, die einen katalytischen Ein-fluss ausübt, wird bevorzugt eine organische Säure zugesetzt. Es kommen niedermolekulare Mono- und Polycarbonsäuren in Betracht, die auch durch gegenüber dem Amin inerte Gruppen substituiert sein können, beispielsweise Hydroxygruppen. Bevorzugt sind niedere Alkancarbonsäuren, insbesondere die Essigsäure, da technisches Diketen ohnehin Acetanhydrid enthält. Im allgemeinen werden bis zu etwa 0,5 Mol Säure pro Mol Amin zugesetzt; grössere Säuremengen sind möglich, bringen jedoch keine Verbesserung mehr.

Die Umsetzungstemperatur soll am Anfang 10°C nicht überschreiten. Um bei vertretbaren Reaktionszeiten den Kühlaufwand möglichst gering zu halten, gibt man das Diketen zweckmässig portionsweise oder stetig, gegebenenfalls mit Unterbrechungen, zu. Das Diketen wird zweckmässig in geringem Überschuss von bis zu etwa 10% eingesetzt. Ein grösserer Überschuss an Diketen kann zu einer Qualitätsverschlechterung des Produkts führen.

Während der Zugabe des Diketens kann man die Temperatur des Reaktionsgemisches auf über Raumtemperatur ansteigen lassen und danach zur Vervollständigung der Reaktion nachrühren.

Die Reaktionsprodukte fallen in kristalliner Form an. Zur Isolierung wird das Reaktionsgemisch zweckmässig gekühlt, vorteilhaft auf Temperaturen von etwa −10° bis −5°C. Aus der Mutterlauge kann man durch Einengen eine weitere Fraktion gewinnen.

Bei dem erfindungsgemässen Verfahren fällt praktisch kein Abwasser an. Das Löse- bzw. Verdünnungsmittel wird durch Destillation wiedergewonnen, wobei zu beachten ist, dass hierbei im Verhältnis zu wasser sehr viel weniger Energie erforderlich ist. Die harzartigen Destillationsrückstände können der Verbrennung zugeführt werden.

In den folgenden Beispielen beziehen sich die Prozentangaben auf das Gewicht.

Beispiel 1

232,6 g technisches, feuchtes 2,5-Dimethoxy-4-chloranilin mit einem reingehalt von 80,6% (entsprechend 187,5 g 100%ige Ware = 1 Mol) werden mit 700 ml Toluol so lange gekocht, bis an einem Wasserabscheider kein Wasser mehr abgetrennt wird. Nach dem Abkühlen gibt man

20 g Ameisensäure (100%ig) und
1 g Wasser zu und lässt dann innerhalb von etwa 30 Minuten
92 g Diketen (1,1 Mol) so zulaufen, dass die Temperatur 50°C nicht übersteigt. Man rührt noch 1 Stunde ohne Kühlung nach, setzt dann
5 g Kohle und
5 g Kieselgur zu und klärt die reaktionslösung bei ca. 100°C. Nach dem Abkühlen auf Raumtemperatur kühlt man zur Vervollständigung der Fällung 2 Stunden mit Sole auf −8°C bis −5°C und saugt das ausgefallene Produkt ab. Nach dem Trocknen erhält man
233,5 g 1 - Acetoacetylamino - 2,5 - dimethoxy - 4 - chlorbenzol (86% d. Th.). Die Mutterlauge wird auf etwa 1/3 eingeengt, wobei weitere
16,3 g Produkt (=6% d.Th.) anfalle.

Das Produkt ird in vorzüglicher Reinheit erhalten. Wird eine Probe mit Methanol extrahiert, so lässt sich im Extrakt praktisch kein Amin mehr nachweisen. Das Produkt ist vorzüglich als Diazokomponente zur Herstellung von Pigmenten geeignet.

In analoger Weise verläuft auch die Umsetzung mit 2,4-Dimethylanilin und 2-Methyl-5-chlor-anilin.

Anstelle der Ameisensäure können mit dem gleichen Ergebnis auch Propionsäure und insbesondere Essigsäure eingesetzt werden.

Beispiel 2

121 g destilliertes 2,4-Dimethylanilin (Reingehalt 99,9%; =0,1 Mol) werden mit
700 ml wasserfreiem Toluol gemischt und mit
25 g Essigsäure, wasserfrei, versetzt. Dazu gibt man
0,3 g Wasser und innerhalb von 30 Minuten ohne Kühlung
92 g Diketen (1,1 Mol).
Dabei steigt die Temperatur allmählich auf 50°C an. Man rührt eine Stunde nach, setzt dann
5 g Kohle und
5 g Kieselgur zu und klärt die Reaktionslösung bei ca. 100°C. Nun kühlt man auf Raumtemperatur (ca. 20°C) und vervollständigt die Fällung durch 2-stündiges Kühlen mit Sole auf −8° bis −5°C. Man saugt das ausgefallene Produkt ab, trocknet und erhält
165 g 1 - Acetoacetylamino - 2,4 - dimethylbenzol (80% d.Th.). Die Mutterlauge wird auf etwa 1/3 eingeengt, wobei weitere
14,5 g Produkt anfallen (=7% d.Th.).

**Patentansprüche**

1. Verfahren zur Herstellung von 1 - Acetoacetylamino - 2,5 - dimethoxy - 4 - chlorbenzol, 1 - Acetoacetylamino - 2,4 - dimethyl - benzol und 1 - Acetoacetylamino - 2 - methyl - 5 - chlorbenzol aus Diketen und dem entsprechenden Anilin in Gegenwart von Wasser unter Zusatz katalytischer Säuremengen, dadurch gekennzeichnet, dass man das Anilin in einem inerten organischen Löse- oder Verdünnungsmittel in Gegenwart von 0,1—6%, bezogen auf das Gewicht des Anilins, Wasser umsetzt.

2. Verfahren nach Anspruch 1, dadurch gekennzeichnet, dass die Wassermenge 0,1 bis 3% beträgt.

3. Verfahren nach Anspruch 1 und 2, dadurch gekennzeichnet, dass die Wassermenge 0,15 bis 0,35% beträgt.

4. Verfahren nach Anspruch 1—3, dadurch gekennzeichnet, dass man als inertes Löse- oder Verdünnungsmittel einen Stoff einsetzt, der mit Wasser ein Azeotrop bildet.

5. Verfahren nach Anspruch 1—4, dadurch gekennzeichnet, dass man als inertes Löse- oder Verdünnungsmittel Toluol oder Xylol einsetzt.

6. Verfahren nach Anspruch 1—5, dadurch gekennzeichnet, dass man ein wasserhaltiges Anilin in situ durch Erhitzen mit einem inerten organischen Löse- oder Verdünnungsmittel, das mit Wasser ein Azeotrop bildet, trocknet und die erforderliche Wassermange zusetzt.

**Claims**

1. Process for the manufacture of 1 - acetoacetylamino - 2,5 - dimethoxy - 4 - chlorobenzene, 1 - acetoacetylamino - 2,4 - dimethylbenzene and 1 - acetoacetylamino - 2 - methyl - 5 - chlorobenzene from diketene and the corresponding aniline in the presence of water with the addition of catalytic amounts of acid, characterized by reacting the aniline in an inert organic solvent or diluent in the presence of from 0.1 to 6%, referred to the weight of the aniline, of water.

2. A process as claimed in claim 1, characterized in that the amount of water is in the range of from 0.1 to 3%.

3. A process as claimed in claims 1 and 2, characterized in that the amount of water is in the range of from 0.15 to 0.35%.

4. A process as claimed in claims 1 to 3, characterized in that the inert solvent or diluent is a substance which forms with water an azeotropic mixture.

5. A process as claimed in claims 1 to 4, characterized in that the inert solvent or diluent is toluene or xylene.

6. A process as claimed in claims 1 to 5, characterized by drying a water-containing aniline in situ by heating it with an inert organic solvent or diluent which forms with water an azeotropic mixture, and adding the amount of water required.

## Revendications

1. Procédé de préparation de l'acétoacétyl-amino-1 diméthoxy - 2,5 chloro - 4 benzène, de l'acétoacétylamino - 1 diméthyl - 2,4 benzène ou de l'acétoacétylamino-1 méthyl-2 chloro-5 benzène à partir du dicétène et de l'aniline correspondante, en présence d'eau et en présence d'une quantité catalytique d'un acide, procédé caractérisé en ce qu'on fait réagir l'aniline dans un solvant ou diluant organique inerte, en présence de 0,1 à 6% d'eau par rapport au poids de l'aniline.

2. Procédé selon la revendication 1, caractérisé en ce que la quantité d'eau est comprise entre 0,1 et 3%.

3. Procédé selon l'une des revendications 1 et 2, caractérisé en ce que la quantité d'eau est comprise entre 0,15 et 0,35%.

4. Procédé selon l'une quelconque des revendications 1 à 3, caractérisé en ce qu'on utilise, comme solvant ou diluant inerte, un corps capable de former un mélange azéotropique avec l'eau.

5. Procédé selon l'une quelconque des revendications 1 à 4, caractérisé en cen qu'on utilise, comme solvant ou diluant inerte, le toluène ou un xylène.

6. Procédé selon l'une quelconque des revendications 1 à 5, caractérisé en ce qu'on sèche une aniline aqueuse in situ par chauffage avec un solvant ou diluant organique inerte capable de former un mélange azéotropique avec l'eau, et on ajoute la quantité d'eau requise.